# EUROPEAN PATENT APPLICATION

(11) **EP 3 187 100 A1**
(43) Date of publication of application: **05.07.2017**
(21) Application number: 15834918.3
(22) Date of filing: 20.08.2015
(51) Int. Cl.: A61B 3/113

(54) **LINE-OF-SIGHT DETECTION DEVICE**

(30) Priority: 29.08.2014 JP 2014176128
(71) Applicant: Alps Electric Co., Ltd., Tokyo 145-8501 (JP)
(72) Inventor: KAWAUCHI, Takahiro, Tokyo 145-8501 (JP); YAMASHITA, Tatsumaro, Tokyo 145-8501 (JP)
(74) Representative: Schmitt-Nilson Schraud Waibel Wohlfrom Patentanwälte Partnerschaft mbB
(86) International application number: PCT/JP2015/073350
(87) International publication number: WO 2016/031666

(57) **Abstract**

There is provided a line-of-sight detection apparatus that acquires, by performing detection-light irradiation once, images for generation of a bright pupil image and a dark pupil image and that consequently realizes a higher-speed line-of-sight detection process. An image acquisition unit acquires images from a plurality of cameras arranged so as to be spaced apart by a predetermined distance such that the optical axes are not substantially coaxial with each other; a plurality of light sources are each arranged such that the optical axis is substantially coaxial with either of the plurality of cameras; and as first image acquisition regarding detection light supplied from a first light source that is one of the plurality of light sources, an image for extracting a bright pupil image is acquired using a camera that is substantially coaxial with the first light source, and an image for extracting a dark pupil image is acquired using a camera that is not substantially coaxial with the first light source.

## Description

### Technical Field

The present invention relates to a line-of-sight detection apparatus capable of detecting the direction of the line of sight of a car driver or other subjects.

### Background Art

In a point-of-gaze detection method described in PTL 1, using two or more cameras and light sources provided on the outside of apertures of these cameras, an image of the face of a subject is generated as a bright pupil image and a dark pupil image; for each camera, a vector from a corneal reflection point of the subject to a pupil thereof on a plane perpendicular to a reference line connecting the camera and the pupil is calculated on the basis of these images; and the direction of a line of sight of the subject with respect to the reference line of the camera is calculated using a predetermined function in accordance with this vector. The point of gaze of the subject can be detected on a predetermined plane by further correcting the function such that the calculated directions of the lines of sight corresponding to the respective cameras become close to each other and by determining the point of an intersection of the lines of sight on the predetermined plane through calculation of the directions of the lines of sight using the corrected function.

In the point-of-gaze detection method described in PTL 1, light emission elements that output light of wavelengths different from each other are provided as the light sources. By causing these light emission elements to alternately emit light, a bright pupil image is generated when an eye of the subject is irradiated with illumination light by one of the light emission elements, and a dark pupil image is generated when the eye of the subject is irradiated with illumination light by the other light emission element.

### Citation List

### Patent Literature

PTL 1: WO 2012/020760

### Summary of Invention

### Technical Problem

However, in the point-of-gaze detection method described in PTL 1, illumination-light irradiation needs to be performed twice to generate a bright pupil image and a dark pupil image; thus, it takes time to acquire a pupil image, which makes it difficult to increase the speed of point-of-gaze or line-of-sight detection processing performed using these pupil images.

The purpose of the present invention is to provide a line-of-sight detection apparatus that can acquire, by performing detection-light irradiation once, images for generation of a bright pupil image and a dark pupil image and that can realize a higher-speed line-of-sight detection process.

### Solution to Problem

In order to solve the above-described problems, a line-of-sight detection apparatus having first and second cameras each for acquiring an image of a region including at least an eye, the first and second cameras being arranged so as to be spaced apart, a first light source that is arranged near the first camera, a second light source that is arranged near the second camera, and a pupil-image extraction unit for extracting a pupil image from a bright pupil image and a dark pupil image acquired by the respective cameras is characterized in that
first image acquisition for causing the first light source to be turned on and for acquiring a bright pupil image using the first camera and a dark pupil image using the second camera and second image acquisition for causing the second light source to be turned on and for acquiring a bright pupil image using the second camera and a dark pupil image using the first camera are performed.

The line-of-sight detection apparatus according to the present invention may include a corneal-reflection-light center detection unit for detecting corneal reflection light from the dark pupil image and a line-of-sight direction calculation unit for calculating a line-of-sight direction of the subject from the pupil image and the corneal reflection light.

The line-of-sight detection apparatus according to the present invention can acquire a bright pupil image and a dark pupil image when one of the first light source and the second light source emits light, and can perform a line-of-sight detection process at high speed.

Preferably, in the line-of-sight detection apparatus according to the present invention, the first camera and the second camera simultaneously acquire images when the first light source stays on, and the first camera and the second camera simultaneously acquire images when the second light source stays on.

The cycle of acquisition of bright and dark pupil images using two cameras can be shortened by simultaneously performing image acquisition for extracting a bright pupil image and image acquisition for extracting a dark pupil image, and the line-of-sight detection process can be performed at higher speed.

Note that, in the present invention, while the first light source stays on, image acquisition using the first camera and image acquisition using the second camera may be alternately performed such that they do not overlap. While the second light source stays on, the image acquisition using the first camera and the image acquisition using the second camera may also be alternately performed such that they do not overlap.

According to the present invention, preferably, the first image acquisition and the second image acquisition are alternately performed.

In this case, preferably, an exposure control unit for monitoring the brightness of the images acquired in the first image acquisition and for controlling, on the basis of the monitoring result, an exposure condition or conditions for acquiring images using the cameras in the next first image acquisition is provided.

In addition, preferably, an exposure control unit for monitoring the brightness of the images acquired in the second image acquisition and for controlling, on the basis of the monitoring result, an exposure condition or conditions for acquiring images using the cameras in the next second image acquisition is provided.

For example, the present invention is provided with an image comparison unit for comparing the brightness of the images acquired in the first image acquisition with the brightness of the images acquired in the second image acquisition, and the exposure control unit for controlling, in accordance with a result of the comparison made by the image comparison unit, the exposure condition or conditions in at least one of the first image acquisition and the second image acquisition.

In this case, the image comparison unit compares the brightness of the image that is acquired in the first image acquisition and that is to be the bright pupil image with the brightness of the image that is acquired in the second image acquisition and that is to be the dark pupil image.

In addition, the image comparison unit compares the brightness of the image that is acquired in the first image acquisition and that is to be the dark pupil image with the brightness of the image that is acquired in the second image acquisition and that is to be the bright pupil image.

Alternatively, the image comparison unit compares the images acquired in the first image acquisition or the second image acquisition with target values.

Variations in the brightness of acquired images due to the differences in brightness or the like between the plurality of light sources can be reduced in the above-described invention. Bright and dark pupil images whose image quality is at a certain level can thus be obtained, thereby enabling high-accuracy line-of-sight detection.

In the line-of-sight detection apparatus according to the present invention, preferably, the exposure condition or conditions are at least one of light emission times and light emission levels of the light sources as well as image acquisition times and gains of the cameras.

In the line-of-sight detection apparatus according to the present invention, preferably, the exposure condition or conditions include image acquisition times of the cameras, and the light emission time of the light source is controlled in accordance with the longer one of the image acquisition time of the first camera and the image acquisition time of the second camera in at least one of the first image acquisition and the second image acquisition.

As a result, complicated control is unnecessary to acquire images for bright pupil images and dark pupil images.

### Advantageous Effects of Invention

According to the present invention, images for generation of a bright pupil image and a dark pupil can be acquired while one of the first light source and the second light source stays on, and thus a higher-speed line-of-sight detection process can be realized.

In addition, according to the present invention, variations in the brightness of acquired images due to the differences in brightness or the like between the light sources can be reduced. Bright and dark pupil images whose image quality is at a certain level can thus be obtained, thereby enabling high-accuracy line-of-sight detection. Brief Description of Drawings
[Fig. 1] Fig. 1 is a front view illustrating the configuration of a line-of-sight detection apparatus according to an embodiment of the present invention.
[Fig. 2] Fig. 2 is a block diagram illustrating the configuration of the line-of-sight detection apparatus according to the embodiment of the present invention.
[Fig. 3] Fig. 3 includes plan views illustrating relationships between line-of-sight directions of an eye of a person and cameras.
[Fig. 4] Fig. 4 includes diagrams for describing calculation of a line-of-sight direction from the center of a pupil and the center of corneal reflection light.
[Fig. 5] Fig. 5 is a chart illustrating image acquisition timings in the line-of-sight detection apparatus according to the embodiment of the present invention.
[Fig. 6] Fig. 6 is a chart illustrating a timing of light emission of a light source and image acquisition timings of cameras.

### Description of Embodiments

In the following, a line-of-sight detection apparatus according to an embodiment of the present invention will be described in detail with reference to the drawings.

### <Configuration of Line-of-sight Detection Apparatus>

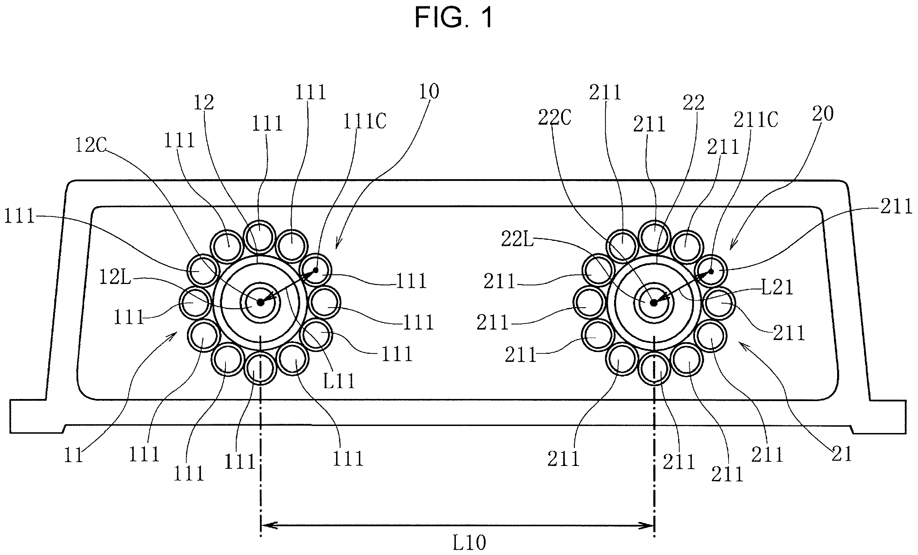

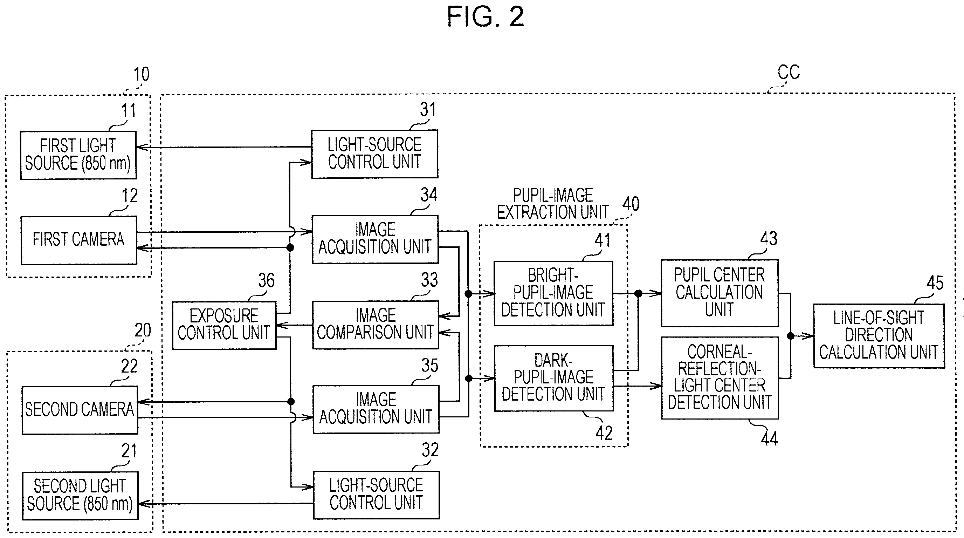

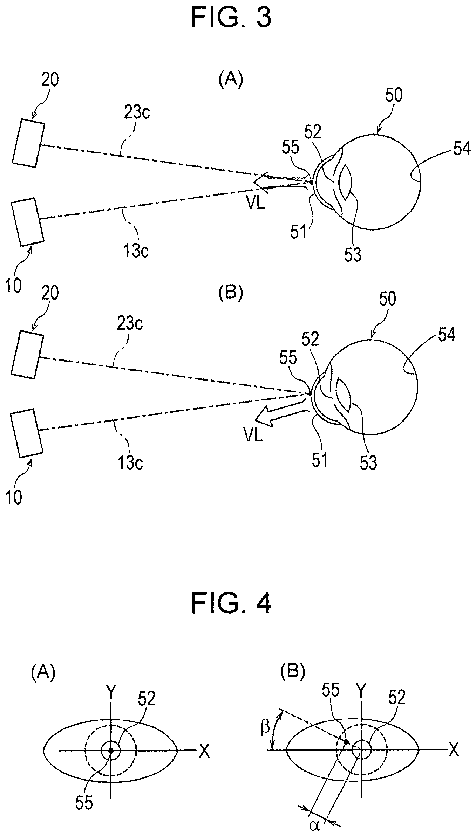

Fig. 1 is a front view illustrating the configuration of the line-of-sight detection apparatus according to the embodiment of the present invention. Fig. 2 is a block diagram illustrating the configuration of the line-of-sight detection apparatus according to the embodiment of the present invention. Fig. 3 includes plan views illustrating relationships between line-of-sight directions of an eye of a person and cameras. Fig. 4 includes diagrams for describing calculation of a line-of-sight direction from the center of a pupil and the center of corneal reflection light.

The line-of-sight detection apparatus according to the present embodiment is provided with, as illustrated in Fig. 2, two image receiving devices 10 and 20 and a computation control unit CC, and is installed inside the cabin of a vehicle and, for example, at an upper portion of the instrument panel, the windshield, or the like, so as to be directed toward the face of the driver as a subject.

As illustrated in Fig. 1 and Fig. 3, the two image receiving devices 10 and 20 are arranged so as to be spaced apart by a predetermined distance L10, and optical axes 13C and 23C of cameras 12 and 22 with which the respective receiving devices 10 and 20 are provided are directed toward an eye 50 of the driver or the like. The cameras 12 and 22 include an image pickup element such as a complementary metal oxide semiconductor (CMOS) or a charge-coupled device (CCD) and acquire images of the face including an eye of the driver. Light is detected by a plurality of pixels arranged two-dimensionally in the image pickup element.

As illustrated in Fig. 1 and Fig. 2, the image receiving device 10 is provided with a first light source 11 and a first camera 12, and the first light source 11 is constituted by 12 light-emitting diode (LED) light sources 111. These LED light sources 111 are arranged in a circular shape on the outer side of a lens 12L of the camera 12 such that optical axes 111C thereof are spaced apart from an optical axis 12C of the camera 12 by a distance L11. The image receiving device 20 is provided with a second light source 21 and a second camera 22, and the second light source 21 is constituted by 12 LED light sources 211. These LED light sources 211 are arranged in a circular shape on the outer side of a lens 22L of the second camera 22 such that optical axes 211C thereof are spaced apart from an optical axis 22C of the second camera 22 by a distance L21.

Preferably, band-pass filters corresponding to the wavelength of detection light emitted by the light sources 11 and 21 are arranged in the first camera 12 and the second camera 22. As a result, the extent of entry of light having wavelengths other than that of the detection light can be reduced, and thus image brightness comparison in an image comparison unit 33, pupil image extraction in a pupil-image extraction unit 40, line-of-sight direction calculation in a line-of-sight direction calculation unit 45 can be performed with high accuracy.

The LED light sources 111 of the first light source 11 and the LED light sources 211 of the second light source 21 emit, for example, infrared light (near-infrared light) having a wavelength of 850 nm as the detection light, and are arranged so that this detection light can be supplied to the eyes of the subject. Here, 850 nm is a wavelength at which the optical absorptance is low within the eyeballs of the eyes of a person, and light of this wavelength tends to be reflected by the retinas at the back of the eyeballs.

In view of the distance between the line-of-sight detection apparatus and the driver as the subject, the distance L11 between the optical axis of the first camera 12 and each of the optical axes of the LED light sources 111 is sufficiently shorter than the distance L10 between the optical axis of the first camera 12 and the optical axis of the second camera 22, and thus the optical axes of the first light source 11 and the first camera 12 can be regarded as axes that are substantially coaxial with each other. Likewise, the distance L21 between the optical axis of the second camera 22 and each of the optical axes of the LED light sources 211 is sufficiently shorter than the distance L10 between the optical axis of the first camera 12 and the optical axis of the second camera 22, and thus the optical axes of the second light source 21 and the second camera 22 can be regarded as axes that are substantially coaxial with each other. Note that the distance L10 between the optical axes is set to, for example, a length that almost matches the distance between both eyes of a person.

In contrast to this, since the distance L10 between the optical axis of the first camera 12 and the optical axis of the second camera 22 is sufficiently long, the optical axes of the first light source 11 and the first camera 12 are not coaxial with the optical axes of the second light source 21 and the second camera 22. Regarding the above-described state, expressions such as "two members are substantially coaxial" or the like may be used, and expressions such as "two members are not coaxial" or the like may be used in the following description.

The computation control unit CC includes a CPU and a memory of a computer, and computation for functions of the blocks illustrated in Fig. 2 is performed by executing preinstalled software.

The computation control unit CC illustrated in Fig. 2 is provided with light-source control units 31 and 32, the image comparison unit 33, image acquisition units 34 and 35, an exposure control unit 36, the pupil-image extraction unit 40, a pupil center calculation unit 43, a corneal-reflection-light center detection unit 44, and the line-of-sight direction calculation unit 45.

The light-source control unit 31 and the light-source control unit 32 control on-off of the first light source 11 and that of the second light source 21, respectively, in accordance with a command signal from the exposure control unit 36. Images acquired by the cameras 12 and 22 are acquired by the respective image acquisition units 34 and 35 on a per-frame basis.

Regarding the images acquired by the image acquisition units 34 and 35, that is, an image for extracting a bright pupil image or a dark pupil image, the image comparison unit 33 compares the brightness of images acquired in first image acquisition and the brightness of images acquired in second image acquisition with each other.

Here, the first image acquisition means a process where images are individually acquired from the first camera 12 and the second camera 22 at the same time or at almost the same time when the detection light is supplied from the first light source 11, and the second image acquisition means a process where images are individually acquired from the first camera 12 and the second camera 22 at the same time or at almost the same time when the detection light is supplied from the second light source 21. Note that the case where the detection light is supplied from the second light source may be first image acquisition, and the case where the detection light is supplied from the first light source may be second image acquisition.

In the image comparison unit 36, an image acquired to obtain a bright pupil image by the first camera 12 in the first image acquisition is compared with, in terms of brightness, an image acquired to obtain a dark pupil image by the first camera 12 in the second image acquisition. In addition, an image acquired to obtain a dark pupil image by the second camera 22 in the first image acquisition is compared with, in terms of brightness, an image acquired to obtain a bright pupil image by the second camera 22 in the second image acquisition. By supplying this comparison result to the exposure control unit 36, the difference in brightness between the bright and dark pupil images acquired by the first camera 12 (the difference in brightness between images of the face excluding pupil portions) can be eliminated or reduced, and the difference in brightness between the dark and bright pupil images acquired by the second camera 22 (the difference in brightness between images of the face excluding pupil portions) can be eliminated or reduced in the first image acquisition and the second image acquisition.

In addition, as an operation of the image comparison unit 36 in another embodiment, an image acquired to obtain a bright pupil image by the first camera 12 in the first image acquisition may be compared with, in terms of brightness, an image acquired to obtain a bright pupil image by the first camera 12 in the next first image acquisition, and an image acquired to obtain a dark pupil image by the second camera 12 in the first image acquisition may also be compared with, in terms of brightness, an image acquired to obtain a dark pupil image by the second camera 12 in the next first image acquisition.

Likewise, images for obtaining bright pupil images may be compared with each other in terms of brightness in the second image acquisition and in the next second image acquisition, and images for obtaining dark pupil images may be compared with each other in terms of brightness in the second image acquisition and in the next second image acquisition. By supplying this comparison result to the exposure control unit 36, bright pupil images and dark pupil images that are not varied in terms of brightness can be obtained in the first image acquisition and in the second image acquisition.

Alternatively, image brightness references may have been determined as target values in advance. In the first image acquisition in the image comparison unit 33, the brightness of an image acquired to obtain a bright pupil image by the first camera 12 is compared with a target value for bright pupil images, and the brightness of an image acquired to obtain a dark pupil image by the second camera 22 is compared with a target value for dark pupil images. The comparison result may also be supplied to the exposure control unit 36. Under this control, exposure conditions for the next first image acquisition are changed so as to optimize the brightness of the image in the previous first image acquisition. The same applies to the second image acquisition.

A comparison between images in terms of brightness or a comparison between the brightness of an image and a target value is, for example, a comparison between the averages of brightness values of the entire images acquired by the image acquisition units 34 and 35 or a comparison between the sums of the brightness values. Alternatively, a comparison may be made using the difference between a maximum brightness value and a minimum brightness value or standard deviations of brightness values may also be compared with each other.

The exposure control unit 36 controls, in accordance with the result of the comparison made by the image comparison unit 33, exposure conditions for capturing images such that the difference in brightness between images to be compared and acquired in the first image acquisition and the second image acquisition falls within a predetermined range.

Regarding the exposure conditions controlled by the exposure control unit 36, for example, at least one of a light emission time and a light emission level of the first light source 11, a light emission time and a light emission level of the second light source 21, an image acquisition time (a camera exposure time) and a sensor gain of the first camera 12, and an image acquisition time (a camera exposure time) and a sensor gain of the second camera 22 is controlled. A signal corresponding to this control is output from the exposure control unit 36 to the light-source control units 31 and 32, the first camera 12, and the second camera 22. The light emission times and light emission levels of the first light source 11 and the second light source 21 are set in accordance with the control signal in the light-source control units 31 and 32. Image acquisition times corresponding to shutter opening times and the sensor gains are set in accordance with the control signal in the first camera 12 and the second camera 22.

The images acquired by the image acquisition units 34 and 35 are loaded into the pupil-image extraction unit 40 on a per-frame basis. The pupil-image extraction unit 40 is provided with a bright-pupil-image detection unit 41 and a dark-pupil-image detection unit 42.

### <Bright Pupil Image and Dark Pupil Image>

Fig. 3 includes plan views schematically illustrating relationships between the line-of-sight directions of the eye 50 of the subject and the cameras. Fig. 4 includes diagrams for describing calculation of a line-of-sight direction from the center of a pupil and the center of corneal reflection light. In Fig. 3(A) and Fig. 4(A), a line-of-sight direction VL of the subject is directed toward the midpoint between the image receiving device 10 and the image receiving device 20. In Fig. 3(B) and Fig. 4(B), the line-of-sight direction VL is directed in the direction of the optical axis 13C of the camera.

The eye 50 has a cornea 51 at the front, and a pupil 52 and a crystalline lens 53 are positioned behind the cornea 51. A retina 54 is present at the rearmost portion.

Infrared light of 850 nm wavelength emitted from the first light source 11 and the second light source 21 has low absorptance within the eyeball, and the light tends to be reflected by the retina 54. Thus, when the first light source 11 is turned on, infrared light reflected by the retina 54 is detected through the pupil 52, and the pupil 52 appears bright in an image acquired by the first camera 12 that is substantially coaxial with the first light source 11. This image is extracted as a bright pupil image by the bright-pupil-image detection unit 41. The same applies to an image acquired by the second camera 22 that is substantially coaxial with the second light source 21 when this is turned on.

In contrast to this, in the case where an image is acquired by the second camera 22 that is not coaxial with the first light source 11 when the first light source 11 is turned on, infrared light reflected by the retina 54 tends not to be detected by the second camera 22 and thus the pupil 52 appears dark. This image is thus extracted as a dark pupil image by the dark-pupil-image detection unit 42. The same applies to an image acquired by the first camera 12 that is not coaxial with the second light source 21 when this is turned on.

In the pupil-image extraction unit 40 illustrated in Fig. 2, the dark pupil image detected by the dark-pupil-image detection unit 42 is subtracted from the bright pupil image detected by the bright-pupil-image detection unit 41, and preferably the images except for the pupil 52 are canceled out and a pupil image signal with which the shape of the pupil 52 appears bright is generated. This pupil image signal is supplied to the pupil center calculation unit 43. The pupil image signal is subjected to image processing and binarized, and an area image that is a portion corresponding to the shape and area of the pupil 52 is calculated in the pupil center calculation unit 43. Furthermore, an ellipse including this area image is extracted, and the point of intersection of the major and minor axes of the ellipse is calculated as the center position of the pupil 52. Alternatively, the center of the pupil 52 is determined from a pupil-image brightness distribution.

In addition, a dark pupil image signal detected by the dark-pupil-image detection unit 42 is supplied to the corneal-reflection-light center detection unit 44. The dark pupil image signal includes a brightness signal based on reflection light that has been reflected from a reflection point 55 of the cornea 51 illustrated in Fig. 3 and Fig. 4.

As illustrated in Fig. 3(A), when either of the light sources stays on, light from the light source is reflected by the surface of the cornea 51, and the light is acquired by both of the first camera 12 and the second camera 22 and is detected by the bright-pupil-image detection unit 41 and the dark-pupil-image detection unit 42. In particular, in the dark-pupil-image detection unit 42, an image of the pupil 52 is relatively dark and thus the reflection light that has been reflected from the reflection point 55 of the cornea 51 is bright and detected as a spot image.

The reflection light from the reflection point 55 of the cornea 51 forms a Purkinje image, and a spot image having a significantly small area is acquired by image pickup elements of the cameras 12 and 22 as illustrated in Fig. 4. In the corneal-reflection-light center detection unit 44, the spot image is subjected to image processing, and the center of the reflection light from the reflection point 55 of the cornea 51 is determined.

A pupil center calculation value calculated by the pupil center calculation unit 43 and a corneal-reflection-light center calculation value calculated by the corneal-reflection-light center detection unit 44 are supplied to the line-of-sight direction calculation unit 45. A line-of-sight direction is detected from the pupil center calculation value and the corneal-reflection-light center calculation value in the line-of-sight direction calculation unit 45.

In the case illustrated in Fig. 3(A), the line-of-sight direction VL of the eye 50 of the person is directed toward the midpoint between the two image receiving devices 10 and 20. Here, the center of the reflection point 55 from the cornea 51 matches the center of the pupil 52 as illustrated in Fig. 4(A). In contrast to this, in the case illustrated in Fig. 3(B), the line-of-sight direction VL of the eye 50 of the person is directed slightly leftward, and thus the center of the pupil 52 and the center of the reflection point 55 from the cornea 51 become misaligned as illustrated in Fig. 4(B).

In the line-of-sight direction calculation unit 45, a direct distance α between the center of the pupil 52 and the center of the reflection point 55 from the cornea 51 is calculated (Fig. 4(B)). In addition, an X-Y coordinate system is set using the center of the pupil 52 as the origin, and a tilt angle β between a line connecting the center of the pupil 52 with the center of the reflection point 55 and the X axis is calculated. Furthermore, the line-of-sight direction VL is calculated from the direct distance α and the tilt angle β.

The above-described pupil image extraction, corneal-reflection-light center detection, and calculation of the line-of-sight direction VL, the calculation being performed using the pupil image extraction and corneal-reflection-light center detection, are performed on the basis of stereo images obtained by the two cameras 12 and 22, and thus the line-of-sight direction VL can be three-dimensionally determined.

### <Image Capturing and Detection Operation>

In the line-of-sight detection apparatus, the first light source 11 is caused to emit light in the first image acquisition, and images are captured by the first camera 12 and the second camera 22 at the same time or almost at the same time during the light emission. The second light source is caused to emit light in the second image acquisition, and images are captured by the first camera 12 and the second camera 22 at the same time or almost at the same time during the light emission.

Relationships among the first image acquisition and the second image acquisition as well as bright pupil image detection and dark pupil image detection are as follows.
(A) First image acquisition: the first light source 11 is turned on,
   (A-1) The first camera 12 performs image acquisition to obtain a bright pupil image.
   (A-2) The second camera 22 performs image acquisition to obtain a dark pupil image.
(B) Second image acquisition: the second light source 12 is turned on,
   (B-1) The second camera 22 performs image acquisition to obtain a bright pupil image.
   (B-2) The first camera 12 performs image acquisition to obtain a dark pupil image.

The above-described (A) first image acquisition and (B) second image acquisition are basically alternately performed.

After performing the first image acquisition once and the second image acquisition once, every time image acquisition is performed, the image comparison unit 33 compares, in terms of brightness, the images acquired in the previous first image acquisition and second image acquisition with each other, and sends out the comparison result to the exposure control unit 36. The exposure control unit 36 having received the comparison result generates, in accordance with the comparison result, a control signal for controlling the exposure conditions of a certain light source such that the brightness of images to be acquired in future image acquisition falls within a predetermined range. This control signal is sent out to the light-source control unit 31 or 32 corresponding to the light source to be used in the next image acquisition, and to the image acquisition units 34 and 35. For example, the light emission time or light emission level is adjusted for the light source, and the exposure time and gain are adjusted for the camera.

In addition, in this embodiment, the first camera acquires a bright pupil image and the second camera acquires a dark pupil image in the first image acquisition. At this point in time, a pupil image is obtained from the bright pupil image and the dark pupil image, and furthermore corneal reflection light is obtained and a line-of-sight direction can be calculated. Likewise, since the first camera acquires a dark pupil image and the second camera acquires a bright pupil image in the second image acquisition, a line-of-sight direction can also be calculated from a pupil image and corneal reflection light at this point in time.

In this manner, the line-of-sight direction can be calculated at both the time of first image acquisition and the time of second image acquisition, and thus the speed of a line-of-sight detection operation can be increased.

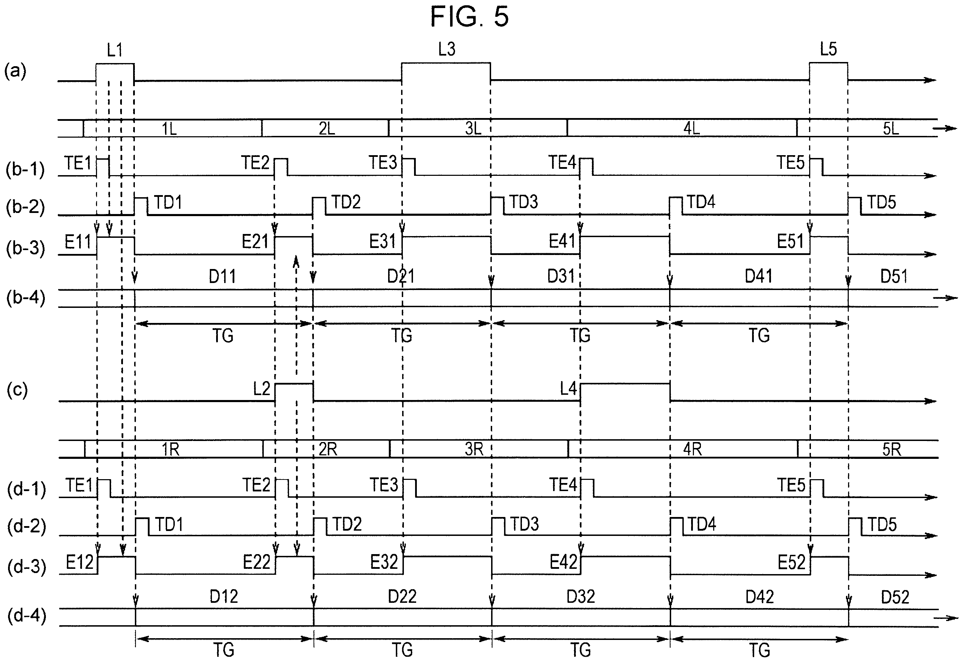

In the following, a more specific description will be given with reference to Fig. 5. Fig. 5 is a chart illustrating image acquisition timings of the line-of-sight detection apparatus according to the present embodiment. In Fig. 5, (a) to (d-4) each indicate a timing of a signal or the like as in the following.
(a): Light emission timings of the first light source 11
(b-1): A trigger signal (TE1, TE2, TE3, TE4, TE5, and so on) for commanding the first camera 12 to start exposure
(b-2): A trigger signal (TD1, TD2, TD3, TD4, TD5, and so on) for commanding starting of image acquisition from the first camera 12 to the image acquisition unit 34
(b-3): Image acquisition (exposure) at the first camera 12
(b-4): Data transfer from the first camera 12 to the image acquisition unit 34
(c): Light emission timings of the second light source 21
(d-1): A trigger signal for commanding the second camera 22 to start exposure
(d-2): A trigger signal for commanding starting of image acquisition from the second camera 22 to the image acquisition unit 35
(d-3): Image acquisition (exposure) at the second camera 22
(d-4): Data transfer from the second camera 22 to the image acquisition unit 35

Here, the timings of the trigger signals of (b-1) and (d-1) are the same. As a result, images are simultaneously acquired from the first camera 12 and the second camera 22. In addition, in units of image acquisition, the light emission time of (a) or (c) and the exposure times of (b-3) and (d-3) are the same in length in the example illustrated in Fig. 5.

In the example illustrated in Fig. 5, as the first image acquisition, exposure E11 at the first camera 12 and exposure E12 at the second camera 22 are simultaneously performed in accordance with light emission L1 of the first light source 11. An image acquired by the first camera 12 that is substantially coaxial with the first light source 11 is an image for bright-pupil-image extraction, and an image acquired by the second camera 22 that is not coaxial with the first light source 11 is an image for dark-pupil-image extraction.

When the light emission L1 ends, the exposure E11 and the exposure E12 end simultaneously. Upon completion of the exposure, data transfer D11 from the first camera 12 to the image acquisition unit 34 and data transfer D12 from the second camera 22 to the image acquisition unit 35 are individually started (the data transfer being more specifically data transfer and frame expansion). The length of time TG of the data transfer D11 and that of the data transfer D12 are the same for respective sections regardless of the exposure times of the light sources 11 and 21.

Next, as the second image acquisition, exposure E21 at the first camera 12 and exposure E22 at the second camera 22 are simultaneously performed in accordance with light emission L2 of the second light source 21. An image acquired by the second camera 22 that is substantially coaxial with the second light source 21 is an image for bright-pupil-image extraction, and an image acquired by the first camera 12 that is not coaxial with the second light source 21 is an image for dark-pupil-image extraction. When the light emission L2 ends, the exposure E21 and the exposure E22 end simultaneously. Upon completion of the exposure, data transfer D21 from the first camera 12 to the image acquisition unit 34 and data transfer D22 from the second camera 22 to the image acquisition unit 35 are individually started. The light emission L1 and L2 as well as the exposure E11, E12, E21, and E22 so far have the same length of time that is preset.

Here, the image comparison unit 33 compares, in terms of brightness, the images acquired in the previous first image acquisition and the second image acquisition with each other, and sends out the comparison result to the exposure control unit 36. In this comparison, the brightness of the image for a bright pupil image and acquired in the first image acquisition is compared with the brightness of the image for a dark pupil image and acquired in the second image acquisition. In addition, the brightness of the image for a dark pupil image and acquired in the first image acquisition is compared with the brightness of the image for a bright pupil image and acquired in the second image acquisition.

Fig. 5 illustrates an example in which the brightness of the image acquired in the first image acquisition based on the light emission L1 of the first light source 11 is lower than the brightness of the image acquired in the second image acquisition based on the light emission L2 of the second light source 21. In the exposure control unit 36, an exposure condition is thus set higher for light emission L3 of the first light source 11 (for example, an image acquisition time (exposure) of the camera is extended) in the first image acquisition for the next period, so that the amount of light to be received is increased. Consequently an image brighter than that acquired in the previous first image acquisition is acquired in the 2nd first image acquisition based on the light emission L3 of the first light source 21.

Furthermore, in the example illustrated in Fig. 5, as a result of comparing the second image acquisition based on the light emission L2 of the second light source 21 with the 2nd first image acquisition based on the light emission L3 of the first light source 11, the exposure condition for image acquisition is corrected to be longer in the 2nd second image acquisition based on light emission L4 of the second light source 21.

Note that, as described above, images acquired in the first image acquisition may be compared with each other in terms of brightness, and the exposure conditions may be changed for the next first image acquisition in accordance with the comparison result; and images acquired in the second image acquisition may be compared with each other in terms of brightness, and the exposure conditions may be changed for the next second image acquisition in accordance with the comparison result.

Alternatively, the images (the bright pupil image and the dark pupil image) acquired in the previous first image acquisition may be compared with predetermined target values (thresholds), and an exposure state may be changed in the next first image acquisition as a result of the comparison. The same applies to the second image acquisition.

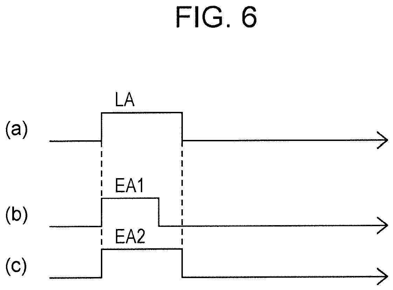

Next, in Fig. 6, (a) indicates a timing of light emission LA of the light source 11 or 21, (b) indicates a timing of exposure EA1 of the first camera 12, and (c) indicates a timing of exposure EA2 of the second camera 22.

In the example illustrated in Fig. 6, an image acquisition time (exposure time) EA1 of the first camera 12 differs from an image acquisition time (exposure time) EA2 of the second camera 22 in image acquisition for which light emission LA of a light source is set by considering, for example, a positional relationship between the two image receiving devices 10 and 20, the difference between light-receiving performance of the camera 12 and that of the camera 22, and the difference in brightness between an image appropriate for extracting a bright pupil image and that appropriate for extracting a dark pupil image.

In this case, the length of the light emission LA of the light source is set to end at the end of the exposure EA21, which is the longer exposure. As a result, complicated control is unnecessary to acquire images for a bright pupil image and a dark pupil image.

In addition, in the above-described embodiment, the detection light from the first light source 11 and the detection light from the second light source 21 have a wavelength of 850 nm; however, if the absorptance within eyeballs is almost at the same level, wavelengths other than this may also be used.

With the configuration above, the following advantages are obtained according to the above-described embodiment.
(1) When, as the first image acquisition or the second image acquisition, detection light is supplied from one of the first light source 11 and the second light source 21, a line-of-sight detection process can be performed at high speed by acquiring an image for extracting a bright pupil image using a camera that is substantially coaxial with the light source and by acquiring an image for extracting a dark pupil image using a camera that is not coaxial with the light source. In addition, the cycle of emission of detection light from the plurality of light sources can be shortened by simultaneously performing image acquisition for extracting a bright pupil image and image acquisition for extracting a dark pupil image, and thus the line-of-sight detection process can be performed at higher speed. Line-of-sight detection can be realized with high accuracy by using a plurality of images for extracting pupil images, the plurality of images being obtained by alternately performing the first image acquisition and the second image acquisition.
(2) With an image comparison unit that compares, in terms of brightness, two images acquired in the first image acquisition with two images acquired in the second image acquisition and an exposure control unit that controls, in accordance with the result of the comparison made by the image comparison unit, exposure conditions of light sources such that the brightness of images to be acquired in the first image acquisition and the second image acquisition falls within a predetermined range, variations in the brightness of acquired images due to the differences in brightness or the like between the plurality of light sources can be reduced. Bright and dark pupil images whose image quality is at a certain level can thus be obtained, thereby enabling high-accuracy line-of-sight detection.

The present invention has been described with reference to the embodiment above; however, the present invention is not limited to the embodiment above and can be improved or changed for improvement or within the scope of the concept of the present invention.

### Industrial Applicability

As described above, the line-of-sight detection apparatus according to the present invention is useful when it is desired that line-of-sight detection be performed with high accuracy and at high speed such as in the case where the line-of-sight detection apparatus is arranged in the cabin of a vehicle and the line of sight of the driver is to be detected.

### Reference Signs List

- 10, 20: image receiving device
- 11: first light source
- 12: first camera
- 12C: optical axis
- 21: second light source
- 22: second camera
- 22C: optical axis
- 33: image comparison unit
- 34, 35: image acquisition unit
- 36: exposure control unit
- 40: pupil-image extraction unit
- 41: bright-pupil-image detection unit
- 42: dark-pupil-image detection unit
- 43: pupil center calculation unit
- 44: corneal-reflection-light center detection unit
- 45: line-of-sight direction detection unit
- 50: eye

## Claims

1. A line-of-sight detection apparatus having first and second cameras each for acquiring an image of a region including at least an eye, the first and second cameras being arranged so as to be spaced apart, a first light source that is arranged near the first camera, a second light source that is arranged near the second camera, and a pupil-image extraction unit for extracting a pupil image from a bright pupil image and a dark pupil image acquired by the respective cameras, the line-of-sight detection apparatus **characterized in that**
first image acquisition for causing the first light source to be turned on and for acquiring a bright pupil image using the first camera and a dark pupil image using the second camera and second image acquisition for causing the second light source to be turned on and for acquiring a bright pupil image using the second camera and a dark pupil image using the first camera are performed.

2. The line-of-sight detection apparatus according to Claim 1, comprising: a corneal-reflection-light center detection unit for detecting corneal reflection light from the dark pupil image; and a line-of-sight direction calculation unit for calculating a line-of-sight direction of the subject from the pupil image and the corneal reflection light.

3. The line-of-sight detection apparatus according to Claim 1 or 2, wherein the first camera and the second camera simultaneously acquire images when the first light source stays on, and the first camera and the second camera simultaneously acquire images when the second light source stays on.

4. The line-of-sight detection apparatus according to any one of Claims 1 to 3, wherein the first image acquisition and the second image acquisition are alternately performed.

5. The line-of-sight detection apparatus according to any one of Claims 1 to 4, provided with an exposure control unit for monitoring the brightness of the images acquired in the first image acquisition and for controlling, on the basis of the monitoring result, an exposure condition or conditions for acquiring images using the cameras in the next first image acquisition.

6. The line-of-sight detection apparatus according to any one of Claims 1 to 5, provided with an exposure control unit for monitoring the brightness of the images acquired in the second image acquisition and for controlling, on the basis of the monitoring result, an exposure condition or conditions for acquiring images using the cameras in the next second image acquisition.

7. The line-of-sight detection apparatus according to any one of Claims 1 to 5, provided with: an image comparison unit for comparing the brightness of the images acquired in the first image acquisition with the brightness of the images acquired in the second image acquisition, and
the exposure control unit for controlling, in accordance with a result of the comparison made by the image comparison unit, the exposure condition or conditions in at least one of the first image acquisition and the second image acquisition.

8. The line-of-sight detection apparatus according to Claim 7, wherein the image comparison unit compares the brightness of the image that is acquired in the first image acquisition and that is to be the bright pupil image with the brightness of the image that is acquired in the second image acquisition and that is to be the dark pupil image.

9. The line-of-sight detection apparatus according to Claim 7 or 8, wherein the image comparison unit compares the brightness of the image that is acquired in the first image acquisition and that is to be the dark pupil image with the brightness of the image that is acquired in the second image acquisition and that is to be the bright pupil image.

10. The line-of-sight detection apparatus according to Claim 7, wherein the image comparison unit compares the images acquired in the first image acquisition or the second image acquisition with target values.

11. The line-of-sight detection apparatus according to any one of Claims 5 to 10, wherein the exposure condition or conditions are at least one of light emission times and light emission levels of the light sources as well as image acquisition times and gains of the cameras.

12. The line-of-sight detection apparatus according to Claim 8, wherein the exposure condition or conditions include image acquisition times of the cameras, and the light emission time of the light source is controlled in accordance with the longer one of the image acquisition time of the first camera and the image acquisition time of the second camera in at least one of the first image acquisition and the second image acquisition.

## Amended claims

### Amended claims under Art. 19.1 PCT

**1.** (Amended) A line-of-sight detection apparatus having first and second cameras each for acquiring an image of a region including at least an eye, the first and second cameras being arranged so as to be spaced apart, a first light source that is arranged near the first camera, a second light source that is arranged near the second camera, and a pupil-image extraction unit for extracting a pupil image from a bright pupil image and a dark pupil image acquired by the respective cameras, the line-of-sight detection apparatus **characterized in that**
first image acquisition for causing the first light source to be turned on and for acquiring an bright pupil image using the first camera and a dark pupil image using the second camera and second image acquisition for causing the second light source to be turned on and for acquiring a bright pupil image using the second camera and a dark pupil image using the first camera are performed, and
the first light source and the second light source emit light of the same wavelength.

**2.** The line-of-sight detection apparatus according to Claim 1, comprising: a corneal-reflection-light center detection unit for detecting corneal reflection light from the dark pupil image; and a line-of-sight direction calculation unit for calculating a line-of-sight direction of the subject from the pupil image and the corneal reflection light.

**3.** The line-of-sight detection apparatus according to Claim 1 or 2, wherein the first camera and the second camera simultaneously acquire images when the first light source stays on, and the first camera and the second camera simultaneously acquire images when the second light source stays on.

**4.** The line-of-sight detection apparatus according to any one of Claims 1 to 3, wherein the first image acquisition and the second image acquisition are alternately performed.

**5.** The line-of-sight detection apparatus according to any one of Claims 1 to 4, provided with an exposure control unit for monitoring the brightness of the images acquired in the first image acquisition and for controlling, on the basis of the monitoring result, an exposure condition or conditions for acquiring images using the cameras in the next first image acquisition.

**6.** The line-of-sight detection apparatus according to any one of Claims 1 to 5, provided with an exposure control unit for monitoring the brightness of the images acquired in the second image acquisition and for controlling, on the basis of the monitoring result, an exposure condition or conditions for acquiring images using the cameras in the next second image acquisition.

**7.** The line-of-sight detection apparatus according to any one of Claims 1 to 5, provided with: an image comparison unit for comparing the brightness of the images acquired in the first image acquisition with the brightness of the images acquired in the second image acquisition, and
the exposure control unit for controlling, in accordance with a result of the comparison made by the image comparison unit, the exposure condition or conditions in at least one of the first image acquisition and the second image acquisition.

**8.** The line-of-sight detection apparatus according to Claim 7, wherein the image comparison unit compares the brightness of the image that is the bright pupil image acquired in the first image acquisition with the brightness of the image that is the dark pupil image acquired in the second image acquisition.

**9.** The line-of-sight detection apparatus according to Claim 7 or 8, wherein the image comparison unit compares the brightness of the image that is the dark pupil image acquired in the first image acquisition with the brightness of the image that is the bright pupil image acquired in the second image acquisition.

**10.** The line-of-sight detection apparatus according to Claim 7, wherein the image comparison unit compares the images acquired in the first image acquisition or the second image acquisition with target values.

**11.** The line-of-sight detection apparatus according to any one of Claims 5 to 10, wherein the exposure condition or conditions are at least one of light emission times and light emission levels of the light sources as well as image acquisition times and gains of the cameras.

**12.** The line-of-sight detection apparatus according to Claim 8, wherein the exposure condition or conditions include image acquisition times of the cameras, and the light emission time of the light source is controlled in accordance with the longer one of the image acquisition time of the first camera and the image acquisition time of the second camera in at least one of the first image acquisition and the second image acquisition.
